# EUROPEAN PATENT SPECIFICATION

(11) **EP 1 300 159 B1**
(45) Date of publication and mention of the grant of the patent: **10.10.2007**
(21) Application number: 01945613.6
(22) Date of filing: 27.06.2001
(51) Int. Cl.: A61K 45/00, A61K 31/4985, A61K 31/437, A61K 31/5025, A61K 31/403, A61K 31/404, A61P 35/00, C07D 487/04, C07D 471/04, C07D 209/86, C07D 405/04

(54) **REMEDIES FOR CANCER**
HEILMITTEL FÜR KREBS
REMEDES CONTRE LE CANCER

(30) Priority: 29.06.2000 JP 2000195434
(43) Date of publication of application: 09.04.2003
(73) Proprietor: Anthera Pharmaceuticals, Inc., San Mateo CA 94403 (US)
(72) Inventor: HANASAKI, Kohji, Shionogi & Co., Ltd., Osaka-shi, Osaka 553-0002 (JP); IKEDA, Minoru, Shionogi & Co., Ltd., Osaka-shi, Osaka 553-0002 (JP); ONO, Takashi, Shionogi & Co., Ltd., Osaka-shi, Osaka 553-0002 (JP)
(74) Representative: Vossius & Partner
(86) International application number: PCT/JP2001/005480
(87) International publication number: WO 2002/000255

(56) References cited:
- EP-A1- 0 675 110
- EP-A1- 0 937 722
- EP-A2- 0 952 149
- WO-A-00/28997
- WO-A-96/40657
- WO-A-99/24026
- WO-A-99/25340
- WO-A1-01/26653
- US-A- 6 028 116
- MURAKAMI M. ET AL.: 'Different functional aspects of the group II subfamily (types IIA and V) and type X secretory phospholipase A(2)s in regulating arachidonic acid release and prostaglandin generation. Implications of cyclooxygenase-2 induction and phospholipid scramblase-mediated cellular membrane perturbation' JOURNAL OF BIOLOGICAL CHEMISTRY vol. 274, no. 44, 29 October 1999, pages 31435 - 31444, XP002945999
- WATANABE K. ET AL.: 'Role of the prostaglandin E receptor subtype EP1 in colon carcinogenesis' CANCER RESEARCH vol. 59, 1999, pages 5093 - 5096, XP002946000
- MORIOKA Y. ET AL.: 'Potential role of group X secretory phospholipase A2 in cyclooxygenase-2-dependent PGE2 formation during colon tumerigenesis' FEBS LETT. vol. 487, no. 2, 29 December 2000, pages 262 - 266, XP002946201

## Description

### Technical Field

The present invention relates to a composition for the prevention or treatment of cancer which contains an inhibitor against type-X sPLA₂ (secretary PLA₂) as an active ingredient.

### Background Art

It is described in Cell. (1996) 87 803-809 that inhibitors of COX-2 at a downstream position of PLA₂ in arachidonate cascade are useful for the treatment of rectosigmoid cancer. As the method of diagnosing tumor including prostate cancer, a quantitative and qualitative assay to detect sPLA₂ proteins or sPLA₂ mRNA levels is described in WO98/05349 (JP Laid-Open (Tokukai) No. 2001/500847). However, neither of the documents describes that compounds having inhibitory activities against type-X sPLA₂ are effective for the treatment of cancer. WO 00/28997A discloses a method for facilitating apoptosis of cancer cells by inhibiting phospholipase activity. WO99/24026 A discloses compositions for the treatment of disorders associated with apoptosis using N-hexerocyclic glyoxylamide compounds.

### Disclosure of Invention

The inventors of the present invention examined the expression of type-X sPLA₂ in various kinds of human pathological tissues with anti-type-X sPLA₂ antibody. They found the elevated expression of type-X sPLA₂ in several tumor tissues.

The immunohistochemical analysis of tumor tissues was performed as follows. At first, anti-human type-X sPLA₂ antibody was added to the slides prepared from normal adult tissues or tumor tissues from cancer patients and incubated for several hours. Next, in order to examine the expression of type-X sPLA₂ in the tissues, the expression of type-X sPLA₂ was visualized by using the methods such as the immunohistochemical labeling to detect the type-X sPLA₂ signals. Consequently, the type-X sPLA₂ signals were detected in the slides prepared from tumor tissues, suggesting that the expression of type-X sPLA₂ is elevated in tumor tissues.

In addition, the inventors of the present invention performed the experiments for neutralization of type-X sPLA₂ signals: Precisely, before the addition of anti-human type-X sPLA₂ antibody to the slides, the slides were incubated with the purified type-X sPLA₂ protein for several hours. Hereafter, the slides were processed with the same procedures as described above to examine the type-X sPLA₂ signals. Consequently, the type-X sPLA₂ signals were disappeared in the slides prepared from tumor tissues.

Thus, the elevated expression of type-X sPLA₂ was confirmed in human tumor tissues.

Furthermore, the inventors of the present invention examined the inhibitory effects of sPLA₂ inhibitor against the type-X sPLA₂-induced release of oleic acid from tumor cell. Consequently, they confirmed that sPLA₂ inhibitors significantly blocked the type-X sPLA₂-induced release of oleic acid from tumor cells.

On the other hand, potential involvement of PGE₂ in the development of tumors has been described in Cancer Research 59, 5093-5096, 1999. Then, the inventors of the present invention examined the inhibitory effects of sPLA₂ inhibitors against the type-X sPLA₂-induced PGE₂ production in tumor cells. Consequently, they confirmed that sPLA₂ inhibitors significantly blocked the type-X sPLA₂-induced PGE₂ production in tumor cells.

The inventors of the present invention achieved the following present invention based on the above experimental results.

That is to say, the present invention relates to the following
I) use of a type -X sPLA₂ inhibitor for the preparation of a medicament for the prevention or treatment of cancer wherein the type-X sPLA₂ inhibitor is a compound represented by the formula: 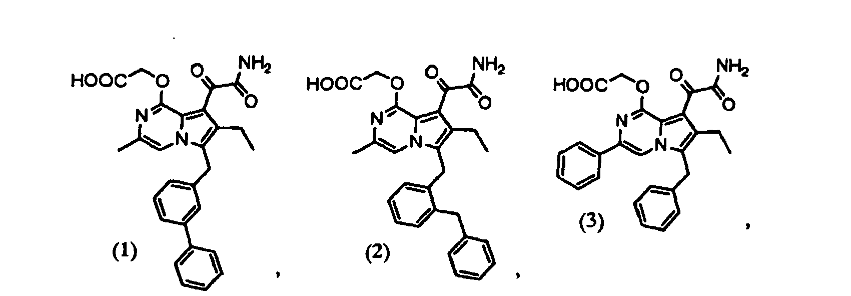 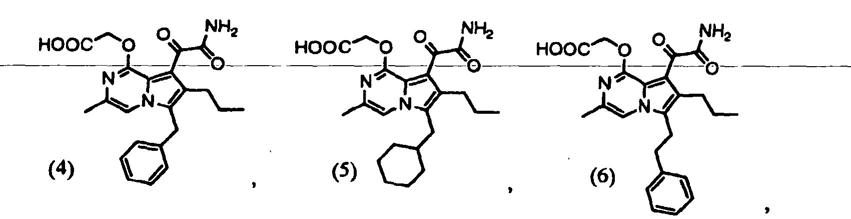 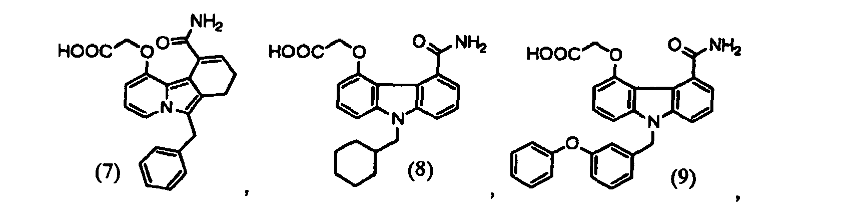 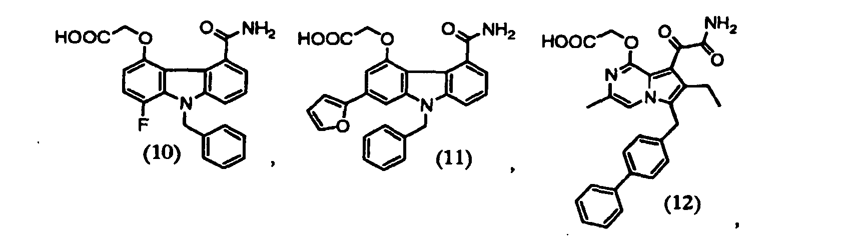 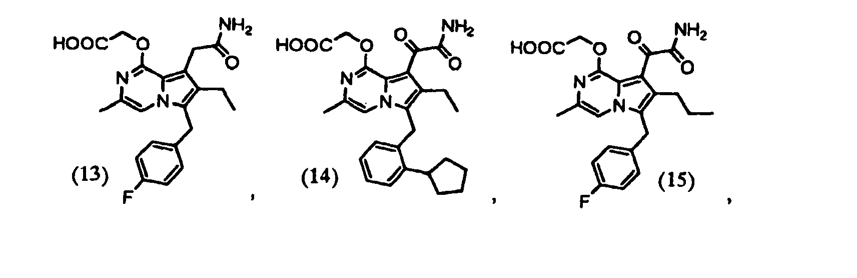 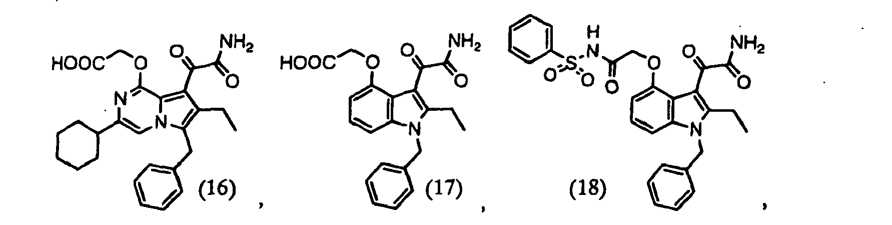 or 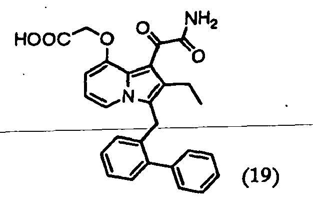 , its pharmaceutically acceptable salt, or its solvate.
II) use according to I) wherein the cancer is colon cancer, lung cancer, liver cancer, gastric cancer, renal cancer, gellbladder cancer, prostatic cancer, pancreatic cancer, testis cancer, ovary cancer or cutaneous cancer.
III) use according to I) wherein the cancer is colon cancer.

The present invention is illustrated in detail as follows

Type-X sPLA₂ inhibitors are compounds which have an inhibitory activity against type-X sPLA₂ and other optional activities such as inhibitory activities against other enzymes or affinities for any receptors. Such inhibitors include any compound having stronger activities against type-X sPLA₂ than that having no such activities in the evaluation test therefore. Especially, type-X sPLA₂ selective inhibitors are preferred as type-X sPLA₂ inhibitors of the present invention. For example, compounds whose IC₅₀ values against type X sPLA₂ are 1 µM or less in the experiment of Example 2 are preferred. Compounds having IC₅₀ values 100 nM or less are more preferred.

A type-X sPLA₂ inhibitor, a compound having type-X sPLA₂ inhibitory activities, having one or more of chiral center(s), may exist as an optically active member. Likewise, a compound containing alkenyl or alkenylene, may be a cis- or trans-isomer. Mixtures of R- and S-isomers as well as of cis- and trans-isomers, and mixtures of R- and S-isomers containing a racemic mixture are included in the scope of the present invention. An asymmetric carbon atom may exist also in a substituent such as alkyl group. All such isomers and mixtures are included in the present invention. A specified stereoisomer can be manufactured by subjecting to stereospecific reaction well known to those skilled in the art applying a previously separated starting material having an asymmetrical center or by preparing a mixture of stereoisomers and separating the mixture in accordance with a well-known manner.

C₁-C₆ alkylesters (e.g. methyl ester, ethyl ester) as prodrugs may also be used.

When a compound having type-X sPLA₂ inhibitory activities has an acidic or basic functional group, a variety of salts having a higher water solubility and more physiologically suitable properties than those of the original compound can be formed. An example of typical pharmaceutically acceptable salts includes salts with alkali metal and alkaline earth metal such as lithium, sodium, potassium, magnesium, aluminum. A salt is easily manufactured from a free acid by either treating an acid in a solution with a base, or allowing an acid to be in contact with an ion exchange resin. Addition salts of the compounds having type-X sPLA₂ inhibitory activities with relatively non-toxic inorganic bases and organic bases, for example, amine cation, ammonium, and quaternary ammonium derived from nitrogenous bases having a basicity sufficient for forming a salt of the compounds of the present invention are included in the definition of "pharmaceutically acceptable salts". (e.g., S. M. Berge et al., "Pharmaceutical Salts, "J. Phar. Sci., 66, 1-19 (1977)). Furthermore, basic groups of a compound having type-X sPLA₂ inhibitory activities are reacted with a suitable organic or inorganic acid to form salts such as acetates, benzenesulfonates, benzoates, bicarbonates, bisulfates, bitartrate, borates, bromides, camsylates, carbonates, chlorides, clavulanates, citrates, edetates, edisylates, estolates, esylates, fluorides, fumarates, gluceptates, gluconates, glutamates, glycolylarsanilates, hexylresorcinates, hydroxynaphthoates, iodides, isothionates, lactates, lactobionates, laurates, malates, maleates, mandelates, mesylates, methylbromides, methylnitrates, methylsulfates, mucates, napsylates, nitrates, oleates, oxalates, palmitates, pantothenates, phosphates, polygalacturonates, salicylates, stearates, subacetates, succinates, tannates, tartrates, tosylates, trifluoroacetates, trifluoromethanesulfonates, valerates and the like.

The solvate includes solvates with organic solvents and/or hydrates. In case of forming a hydrate, a questioned compound may be coordinated with a suitable number of water molecules.

The term "pharmaceutically acceptable" means that carriers, diluents, or additives are compatible with other ingredients in a formulation and are not harmful for recipients.

The "cancer" means various malignant tumors originated from epithelial cells in various tissues, cells such as colon cancer, lung cancer, liver cancer, gastric cancer, renal cancer, gallbladder cancer, prostate cancer, pancreatic cancer, testis cancer, ovarian cancer, cutaneous cancer, esophagus cancer, laryngeal cancer, breast cancer or uterine cancer and exemplified. Based on the experiments, the inventor of the present invention confirmed the elevated expression of type-X sPLA₂ in various tumor tissues, including colon cancer, lung cancer, liver cancer, gastric cancer, renal cancer, gallbladder cancer, prostate cancer, pancreatic cancer, testis cancer, ovarian cancer or cutaneous cancer. Especially, the present invention is useful for the prevention or treatment of colon cancer, lung cancer, liver cancer, gastric cancer, renal cancer, gallbladder cancer, prostate cancer, pancreatic cancer, testis cancer, ovarian cancer or cutaneous cancer.

The term "acidic group" in the present specification means an organic group functioning as a proton donor capable of hydrogen bonding when attached to a basic skeleton through a suitable linking atom (hereinafter defined as "acid linker").

### Best Mode for Carrying Out the Invention

The present invention relates to the prevention or treatment of cancer by a type-X sPLA₂ inhibitor. The type-X sPLA₂ inhibitors may be known ones and are selected from sPLA2 inhibitors defined in claim 1.

Further, the compounds may be identified as type-X sPLA₂ inhibitors by the following procedure.

At first, a cell expressing human type-X sPLA₂ is prepared. That is, cDNA sequence encoding human type-X sPLA₂ (Cupillard et al., J. Biol. Chem, 1997, 272, 15745-15752) is inserted into an expression vector for mammalian cells. The obtained expression vector is transfected into the host cell and the cell stably expressing human type-X sPLA₂ is obtained.

Next, the above-mentioned transfected cell is cultured in medium and its culture supernatant is used for the measurement of each enzyme activity. In order to identify and evaluate an inhibitor of type-X sPLA₂, the following chromogenic assay is utilized. A general explanation for this assay is described in "Analysis of Human Synovial Fluid Phospholipase A2 on Short Chain Phosphatidylcholine-Mixed Micelles: Development of a Spectrophotometric Assay Suitable for a Micortiterplate Reader" (Analytical Biochemistry, 204, pp 190-197, 1992 by Laure. J. Reynolds. Lori L. Hughes and Edward A. Dennis.

Several compounds of the present invention can be synthesized in accordance with the methods described in PCT/JP00/07024, EP-620214 (JP Laid-Open (Tokukai) No. 95/010838, US-5578634), EP-620215 (JP Laid-Open (Tokukai) No. 95/025850, US-5684034), EP-675110 (JP Laid-Open (Tokukai) No. 95/285933, US-5654326), WO 96/03120 (JP Laid-Open No. 98/505336), WO 96/03383 (JP Laid-Open No. 98/505584), WO 98/18464 (EP839806), WO99/51605, WO99/59999.

The composition used for treatment or prevention of cancer in accordance with the present invention may be administered to a patient through a variety of routes including oral, aerosol, rectal, percutaneous, subcutaneous, intravenous, intramuscular, and nasal routes. A formulation according to the present invention may be manufactured by combining (for example, admixing) a curatively effective amount of a compound of the present invention with a pharmaceutically acceptable carrier or diluent. The formulation of the present invention may be manufactured with the use of well-known and easily available ingredients in accordance with a known method.

In case of manufacturing a composition of the present invention, active ingredients are admixed or diluted with a carrier, or they are contained in a carrier in the form of capsule, sacheier, paper, or another container. In case of functioning a carrier as a diluent, the carrier is a solid, semi-solid, or liquid material which functions as a medium. Accordingly, a formulation according to the present invention may be produced in the form of tablet, pill, powder medicine, intraoral medicine, elixir agent, suspending agent, emulsifier, dissolving agent, syrup agent, aerosol agent (solid in liquid medium), and ointment. Such a formulation may contain up to 10% of an active compound. It is preferred to formulate a compound having activities for the treatment or prevention of cancer prior to administration.

Any suitable carrier well known to those skilled in the art may be used for the formulation. In such formulation, a carrier is in the form of solid, liquid, or a mixture thereof. For instance, a compound having type-X sPLA₂ inhibitory activities is dissolved into 4% dextrose/0.5% sodium citrate aqueous solution so as to be 2 mg/mL concentration for intravenous injection. Solid formulation includes powder, tablet, and capsule. Solid carrier consists of one or more of material(s) for serving also as fragrant, lubricant, dissolving agent, suspension, binder, tablet disintegrator, capsule. A tablet for oral administration contains a suitable excipient such as calcium carbonate, sodium carbonate, lactose, calcium phosphate together with a disintegrator such as corn starch, alginic acid and/or a binder such as gelatin, acacia, and a lubricant such as magnesium stearate, stearic acid, talc.

In a powder medicine, a carrier is a finely pulverized solid which is blended with finely pulverized active ingredients. In a tablet, active ingredients are admixed with a carrier having required binding power in a suitable ratio, and it is solidified in a desired shape and size. Powder medicine and tablet contain about 1 to about 99% by weight of the active ingredients being novel compounds according to the present invention. An example of suitable solid carriers includes magnesium carbonate, magnesium stearate, talc, sugar, lactose, pectin, dextrin, starch, gelatin, tragacanth gum, methyl cellulose, sodium carboxymethylcellulose, low-melting wax, and cocoa butter.

An axenic liquid formulation contains suspending agent, emulsifier, syrup agent, and elixir agent. Active ingredients may be dissolved or suspended into a pharmaceutically acceptable carrier such as sterile water, a sterile organic solvent, a mixture thereof and the like. Active ingredients may be dissolved frequently into a suitable organic solvent such as propylene glycol aqueous solution. When finely pulverized active ingredients are dispersed into aqueous starch, sodium carboxymethylcellulose solution, or suitable oil, the other compositions can be prepared.

The dosage varies with the conditions of the disease, administration route, age and body weight of patient. In the case of intravenous administration, the dosage can generally be between 0.01 to 10 mg/kg/h for adult, preferably 0.1 to 1 mg/kg/h.

### Example

### Example 1 Preparation of cells expressing human type-X sPLA₂ and their culture supernatants

cDNA sequence encoding human type-X sPLA₂ (Cupillard et al., J. Biol. Chem, 1997, 272, 15745-15752) was inserted into the downstream region of the promoter of pSVL SV40 Late Promoter Expression Vector (Amersham Pharmacia Biotech Inc.) that is an expression vector for mammalian cells. The obtained expression vector was transfected into the host CHO cells with a LipofectAMINE reagent (Gibco BRL Inc.) according to the manufacture's instruction to obtain the CHO cells stably expressing human type-X sPLA₂. The transfected cell was cultured in α-MEM medium containing 10% fetal calf serum for 3 days and its culture supernatant was used for the measurement of each enzyme activity.

### Example 2 Inhibition test

In order to identify and evaluate an inhibitor of type·X sPLA₂. the following chromogenic assay is utilized. This assay has been applied for high volume screening using a 96-well microtiterplate. A general explanation for this assay is described in "Analysis of Human Synovial Fluid Phospholipase A2 on Short Chain Phosphatidylcholine-Mixed Micelles: Development of a Spectrophotometric Assay Suitable for a Micortiterplate Reader" (Analytical Biochemistry, 204, pp 190-197, 1992 by Laure. J. Reynolds. Lori L. Hughes and Edward A. Dennis.

Test compounds (or solvent blank) were added according to the alignment of plates that had been previously set. Human type-X sPLA₂ was incubated (30 min at 40 °C (15 µl/well)) with diheptanoylthio PC (1 mM) in the presence of Triton X-100 (0.3 mM) and 5,5'-dithiobis(2-nitrobenzoic acid) (125 µM) in Tris-HCl buffer (25 mM, pH 7.5) containing CaCl₂ (10 mM), KCI (100 mM), and bovine serum albumin (1.0 mg/mL). The changes in the absorbance at 405 nm were measured and the inhibition activities were calculated.

The IC₅₀ value was determined by plotting the log concentration of the above-mentioned compounds (1)-(19) with respect to their inhibition values within 10% to 90% inhibitory range.

Results of the type-X sPLA₂ inhibition test is shown in the following Table 1.

**Table 1**

| Compound No. | IC₅₀(nM) | Compound No. | IC₅₀(nM) |
|---|---|---|---|
| 1 | 10 | 11 | 10 |
| 2 | 10 | 12 | 16 |
| 3 | 5 | 13 | 19 |
| 4 | 27 | 14 | 9 |
| 5 | 12 | 15 | 17 |
| 6 | 17 | 16 | 7 |
| 7 | 5 | 17 | 12 |
| 8 | 3 | 18 | 16 |
| 9 | 13 | 19 | 26 |
| 10 | 12 | | |

### Example 3 Immunohistochemical analysis in human colon cancer tissues with anti-type-X sPLA₂ antibody

In this experiment, anti-type-X sPLA₂ antibody that was described in "The Journal of Biological Chemistry Vol. 274, No. 48, pp.34203-34211 1999" was used. Paraffin embedded preparations of human colon cancer tissues and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200 µg/mL diaminobenzidine hydrochloride substrate dissolved in 50 mmol/L Tris-HCl (pH 7.6) containing 0.006% H₂O₂ for 10 min resulting in the appearance of color dependent on the peroxidase activity to visualize the type-X sPLA₂ expression in the tissue preparations. In addition, the nuclei were counterstained with 0.4% hematoxylin solution. Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60 µg/mL) for 2 hr before the addition to the slides.

Consequently, positive signals representative for type-X sPLA₂ expression were not obviously detected in normal colon, but strongly detected in the tumor cells in human colon adenocarcinoma tissues. Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human colorectal cancer tissues.

### Example 4 Immunohistochemical analysis in human lung cancer tissues with anti-type-X sPLA₂ antibody

In this experiment, anti-type-X sPLA₂ antibody which was described in "The Journal of Biological Chemistry Vol. 274, No. 48, pp.34203-34211 1999" was used. Paraffin embedded preparations of human lung cancer tissues and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200 µg/mL diaminobenzidine hydrochloride substrate dissolved in 50 mmol/L Tris-HCl (pH 7.6) containing 0.006% H₂O₂ for 10 min resulting in the appearance of color dependent on the peroxidase activity to visualize the type-X sPLA₂ expression in the tissue preparations. In addition, the nuclei were counterstained with 0.4% hematoxylin solution. Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60 µg/mL) for 2 hr before the addition to the slides.

Consequently, positive signals representative for type-X sPLA₂ expression were weakly observed in type II pneumocytes in normal human lung. In contrast, the positive signals were strongly detected in tumor cells in the lung tissues prepared from patients of lung cancer. Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human lung cancer tissues.

### Example 5 Immunohistochemical analysis in human liver cancer tissues with anti-type-X sPLA₂ antibody

In this experiment, anti-type-X sPLA₂ antibody which was described in "The Journal of Biological Chemistry Vol. 274, No. 48, pp.34203-34211 1999" was used. Paraffin embedded preparations of human liver cancer tissues and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200 µg/mL diaminobenzidine hydrochloride substrate dissolved in 50 mmol/L Tris-HCl (pH 7.6) containing 0.006% H₂O₂ for 10 min resulting in the appearance of color dependent on the peroxidase activity to visualize the type-X sPLA₂ expression in the tissue preparations. In addition, the nuclei were counterstained with 0.4% hematoxylin solution. Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60 µg/mL) for 2 hr before the addition to the slides.

Consequently, positive signals representative for type-X sPLA₂ expression were weakly observed in hepatic lobule and Kupffer's satellate cells in normal human liver. In contrast, the positive signals were strongly detected in tumor cells in the liver tissues prepared from patients of liver cancer. Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human liver cancer tissues.

### Example 6 Immunohistochemical analysis in human gastric cancer tissues with anti-type-X sPLA₂ antibody

In this experiment, anti-type-X sPLA₂ antibody which was described in "The Journal of Biological Chemistry Vol. 274, No. 48, pp.34203-34211 1999" was used. Paraffin embedded preparations of human gastric cancer tissues and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200 µg/mL diaminobenzidine hydrochloride substrate dissolved in 50 mmol/L Tris-HCl (pH 7.6) containing 0.006% H₂O₂ for 10 min resulting in the appearance of color dependent on the peroxidase activity to visualize the type-X sPLA₂ expression in the tissue preparations. In addition, the nuclei were counterstained with 0.4% hematoxylin solution. Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60µg/mL) for 2 hr before the addition to the slides.

Consequently, positive signals representative for type-X sPLA₂ expression were not obviously detected in normal gaster, but strongly detected in tumor cells in the gastric tissues prepared from patients of gastric cancer. Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human gastric cancer tissues.

### Example 7 Immunohistochemical analysis in human renal cancer tissues with anti-type-X sPLA₂ antibody.

In this experiment, anti-type-X sPLA₂ antibody which was described in "The Journal of Biological Chemistry Vol. 274, No. 48, pp.34203-34211 1999" was used. Paraffin embedded preparations of human renal cancer tissues and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200µg/mL diaminobenzidine hydrochloride substrate dissolved in 50 mmol/L Tris-HCl (pH 7.6) containing 0.006% H₂O₂ for 10 min resulting in the appearance of color dependent on the peroxidase activity to visualize the type-X sPLA₂ expression in the tissue preparations. In addition, the nuclei were counterstained with 0.4% hematoxylin solution. Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60 µg/mL) for 2 hr before the addition to the slides.

Consequently, positive signals representative for type-X sPLA₂ expression were weakly observed in glomerular mesangial cells in normal kidney. In contrast, the positive signals were strongly detected in tumor cells in the kidney prepared from patients of renal cancer. Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human renal cancer tissues.

### Example 8 Immunohistochemical analysis in human gallbladder cancer tissues with anti-type-X sPLA₂ antibody

In this experiment, anti-type-X sPLA₂ antibody which was described in "The Journal of Biological Chemistry Vol. 274, No. 48, pp.34203-34211 1999" was used. Paraffin embedded preparations of human gallbladder cancer tissues and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200 µg/mL diaminobenzidine hydrochloride substrate dissolved in 50 mmol/L Tris-HCl (pH 7.6) containing 0.006% H₂O₂ for 10 min resulting in the appearance of color dependent on the peroxidase activity to visualize the type-X sPLA₂ expression in the tissue preparations. In addition, the nuclei were counterstained with 0.4% hematoxylin solution. Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60 µg/mL) for 2 hr before the addition to the slides.

Consequently, positive signals representative for type-X sPLA₂ expression were not obviously detected in normal gallbladder tissues, but strongly detected in tumor cells in the gallbladder tissues prepared from patients of gallbladder cancer. Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human gallbladder cancer tissues.

### Example 9 Immunohistochemical analysis in human prostate cancer tissues with anti-type-X sPLA₂ antibody

In this experiment, anti-type-X sPLA₂ antibody which was described in "The Journal of Biological Chemistry Vol. 274, No. 48, pp.34203-34211 1999" was used. Paraffin embedded preparations of human prostate cancer tissues and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200 µg/mL diaminobenzidine hydrochloride substrate dissolved in 50 mmol/L Tris-HCl (pH 7.6) containing 0.006% H₂O₂ for 10 min resulting in the appearance of color dependent on the peroxidase activity to visualize the type-X sPLA₂ expression in the tissue preparations. In addition the nuclei were counterstained with 0.4% hematoxylin solution. Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60 µg/mL) for 2 hr before the addition to the slides.

Consequently, positive signals representative for type-X sPLA₂ expression were not obviously detected in normal prostates, but strongly detected in tumor cells in the prostate tissues prepared from patients of prostate cancer. Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human prostate cancer tissues.

### Example 10 Immunohistochemical analysis in human pancreatic cancer tissues with anti-type-X sPLA₂ antibody

In this experiment, anti-type-X sPLA₂ antibody which was described in "The Journal of Biological Chemistry Vol. 274, No. 48, pp.34203-34211 1999" was used. Paraffin embedded preparations of human pancreatic cancer tissues and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200 µg/mL diaminobenzidine hydrochloride substrate dissolved in 50 mmol/L Tris.HCl (pH 7.6) containing 0.006% H₂O₂ for 10 min resulting in the appearance of color dependent on the peroxidase activity to visualize the type-X sPLA₂ expression in the tissue preparations. In addition, the nuclei were counterstained with 0.4% hematoxylin solution. Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60 µg/mL) for 2 hr before the addition to the slides.

Consequently, positive signals representative for type-X sPLA₂ expression were not obviously detected in normal pancreas, but strongly detected in tumor cells in the pancreas prepared from patients of pancreatic cancer. Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human pancreatic cancer tissues.

### Example 11 Immunohistochemical analysis in human testis cancer tissues with anti-type-X sPLA₂ antibody

In this experiment, anti-type-X sPLA₂ antibody which was described in "The Journal of Biological Chemistry Vol. 274, No. 48, pp.34203-34211 1999" was used. Paraffin embedded preparations of human testis cancer tissues and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200 µg/mL diaminobenzidine hydrochloride substrate dissolved in 50 mmol/L Tris-HCl (pH 7.6) containing 0.006% H₂O₂ for 10 min resulting in the appearance of color dependent on the peroxidase activity to visualize the type-X sPLA₂ expression in the tissue preparations. In addition, the nuclei were counterstained with 1% methyl green dye in 0.1 mol/L sodium acetate (pH 4.0). Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60 µg/mL) for 2 hr before the addition to the slides.

Consequently, positive signals representative for type-X sPLA₂ expression were weakly observed in some parts of the seminiferous tubules of normal testis. In contrast, the positive signals were strongly detected in malignant cells present in the seminiferous tubules and/or the epithelium of seminal vesicles in the testis prepared from patients of testis cancer. Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human testis cancer tissues.

### Example 12 Immunohistochemical analysis in human ovarian cancer tissues with anti-type-X sPLA₂ antibody

In this experiment, anti-type-X sPLA₂ antibody which was described in "The Journal of Biological Chemistry Vol. 274, No. 48, pp.34203-34211 1999" was used. Paraffin embedded preparations of human ovarian cancer tissues and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200 µg/mL diaminobenzidine hydrochloride substrate dissolved in 50 mmol/L Tris-HCl (pH 7.6) containing 0.006% H₂O₂ for 10 min resulting in the appearance of color dependent on the peroxidase activity to visualize the type-X sPLA₂ expression in the tissue preparations. In addition, the nuclei were counterstained with 1% methyl green dye in 0.1 mol/L sodium acetate (pH 4.0). Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60 µg/mL) for 2 hr before the addition to the slides.

Consequently, positive signals representative for type-X sPLA₂ expression were not obviously detected in normal ovary, but strongly detected in the malignant cells present in the epithelium of ovarian follicles and/or oviducts in the ovary prepared from patients of ovarian cancer. Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human ovarian cancer tissues.

### Example 13 Immunohistochemical analysis in human cutaneous cancer tissues with anti-type-X sPLA₂ antibody

In this experiment, anti-type-X sPLA₂ antibody which was described in "The Journal of Biological Chemistry Vol. 274, No. 48, pp.34203-34211 1999" was used. Paraffin embedded preparations of human cutaneous cancer tissues (malignant melanoma) and corresponding normal tissues were purchased from Biochain Inc. (San Leandro, CA). The tissue sections in the slides were dewaxed, treated in methanol containing 0.3% H₂O₂ for 30 min to remove the endogenous peroxidase activity and incubated with 5% normal goat serum for 20 min. The slides were then incubated with anti-type-X sPLA₂ antibody (6 µg/mL) in PBS containing 0.1% bovine serum albumin for 14 hr at 4°C. After washing with PBS, they were incubated with biotin-conjugated goat anti-rabbit IgG antibody for 30 min followed by treatment with peroxidase labelled avidin-biotin complex reagent (Vector Laboratories). After washing, the samples were processed with 200 µg/mL diaminobenzidine hydrochloride substrate dissolved in 50 mmol/L Tris-HCl (pH 7.6) containing 0.006% H₂O₂ for 10 min resulting in the appearance of color dependent on the peroxidase activity to visualize the type-X sPLA₂ expression in the tissue preparations. In addition, the nuclei were counterstained with 1% methyl green dye in 0.1 mol/L sodium acetate (pH 4.0). Positive signals representative for type-X sPLA₂ expression was visualized as a dark-brownish color of diaminobenzidine deposit. The neutralization of type-X sPLA₂ specific signals was conducted by incubating anti-type-X sPLA₂ antibody with purified type-X sPLA₂ protein (60 µg/mL) for 2 hr before the addition to the slides.

Consequently, positive signals representative for type-X sPLA₂ expression were weakly observed in the melanocytes of stratum spinosum and/or stratum basale in skin epidermis in normal human cutis. In contrast, the positive signals were strongly detected in hypertrophied melanocytes in the cutis prepared from patients of cutaneous cancer (malignant melanoma). Since the addition of type-X sPLA₂ protein resulted in abolishment of the signals, they were verified as the specific signals for type-X sPLA₂. In addition, there was no positive signal when IgG prepared from non-immunized rabbit was used. Taken together, these findings suggest that the expression of type-X sPLA₂ protein was greatly elevated in human cutaneous cancer (malignant melanoma) tissues.

### Example 14 Effects of sPLA₂ inhibitors on human type-X sPLA₂-induced oleic acid release in human colon carcinoma cell lines, HT-29 cells

Compound (1) and compound (19) was used as test compounds.

Human colon carcinoma cell lines, HT-29 cells (obtained from ATCC) were cultured in DMEM supplemented with 10% fetal calf serum. The cells were washed by phosphate-buffered saline (PBS), detached from culture plates by treatment with trypsin/EDTA solution and further washed by PBS. The resulting cells were resuspended in Hanks' buffered saline containing 0.1% bovine serum albumin (BSA) at a density of 12.5 x 10⁶ cells/mL. Aliquots of the cell suspension (0.4 mL) were transferred into polypropylene tubes and test compounds dissolved in DMSO solution (final concentration; 10 µM) were added. After preincubation for 10 min at 37°C, 100 nM purified human type-X sPLA₂ enzyme (Hanasaki et al. J. Biol. Chem. (1999) 274. 34203-34211) was added (final volume of 0.5 mL). After incubation for 30 min at 37°C, the reaction was stopped by the addition of 2 mL Dole's reagent (heptane : 2-propanol : 1 M H₂SO₄ = 10 : 40 : 1, v/v/v). According to the method of Tojo et al. (J. lipid Res. (1993) 34, 837-844), the released fatty acids were extracted, labeled with 9-anthryldiazomethane (Funakoshi Co.), and the oleic acid was quantified by reverse-phase high performance chromatography (LiChroCART 125-4 Superspher 100 RP-18 column (Merck). From each data, the value in the absence of type-X sPLA₂ was subtracted. The amount of released oleic acid in the presence of each test compound was expressed as the percentage of the increased content by the addition of type-X sPLA₂ enzyme. As shown in Table 2, each test compound significantly inhibited the type-X sPLA₂ -induced oleic acid release.

### Example 15 Effects of sPLA₂ inhibitors on type-X sPLA₂-induced PGE₂ production in human colon carcinoma cell lines. HT-29 cells

HT-29 cells were seeded into 24-well plates at a density of 2.5×10⁵ cells/well. After incubation for 24 h, the cells were washed with PBS and incubated with 30 ng/mL of Tumor necrosis factor-α (R&D Systems, Inc.) in DMEM medium supplemented with 10% fetal bovine serum for 18 h. After washing with PBS, the cells were preincubated with or without test compounds (at a final concentration of 10 µM ; dissolved in DMSO) in Hanks' buffered saline containing 0.1% BSA for 10 min at 37°C, and then stimulated with 100 nM purified human type-X sPLA₂ enzyme in a final volume of 0.5 mL. After incubation for 3 h at 37°C, the culture supernatant was collected following the centrifugation for the removal of floating cells, and its PGE₂ content was quantified with an enzyme-immunoassay kit (Cayman Chemicals Co.). From each data, the value in the absence of type-X sPLA₂ was subtracted. The amount of PGE₂ in the presence of each test compound was expressed as the percentage of the PGE₂ content produced by the addition of type-X sPLA₂ enzyme. As shown in Table 2, each test compound significantly blocked the type-X sPLA₂-induced PGE₂ production.

Potential involvement of PGE₂ in the progression of tumors has been described in Cancer Research 59, 5093-5096, 1999. Since the compounds in the present invention significantly block the type-X sPLA₂-induced PGE₂ production, they can be applied as antitumor agents.

**Table 2**

| Compound (10 µM) | Oleic acid release | PGE₂ production |
|---|---|---|
| No treatment group | 100±2.8 | 100±22.7 |
| (1) | -3.0±3.9** | -4.1±12.6** |
| (19) | 1.8±4.6** | 42.1±9.7** |

| | | |
|---|---|---|
| Student's t-test (*P < 0.05, **P < 0.01) | | |

### Formulation Example

It is to be noted that the following Formulation Examples 1 to 8 are mere illustration of the invention. The term "active ingredient" means the compounds having an inhibitory activity against type-X PLA₂, the prodrugs thereof, their pharmaceutical acceptable salts, or their hydrate as defined in claim 1.

### Formulation Example 1

Hard gelatin capsules are prepared using of the following ingredients:

| | Dose |
|---|---|
| | (mg/capsule) |
| Active ingredient | 250 |
| Starch, dried | 200 |
| Magnesium stearate | 10 |
| Total | 460 mg |

### Formulation Example 2

A tablet is prepared using of the following ingredients:

| | Dose |
|---|---|
| | (mg/tablet) |
| Active ingredient | 250 |
| Cellulose, microcrystals | 400 |
| Silicon dioxide, fumed | 10 |
| Stearic acid | 5 |
| Total | 665 mg |

The components are blended and compressed to form tablets each weighing 665 mg.

### Formulation Example 3

An aerosol solution is prepared containing the following components:

| | Weight |
|---|---|
| Active ingredient | 0.25 |
| Ethanol | 25.75 |
| Propellant 22 (chlorodifluoromethane) | 74.00 |
| Total | 100.00 |

The active compound is mixed with ethanol and the admixture added to a portion of the propellant 22, cooled to -30 °C and transferred to filling device. The required amount is then fed to stainless steel container and diluted with the reminder of the propellant. The valve units are then fitted to the container.

### Formulation Example 4

Tablets, each containing 60 mg of active ingredient, are made as follows.

| | |
|---|---|
| Active ingredient | 60 mg |
| Starch | 45 mg |
| Microcrystals cellulose Polyvinylpyrrolidone (as 10% solution in water) | 35 mg 4 mg |
| Sodium carboxymethyl starch | 4.5 mg |
| Magnesium stearate | 0.5 mg |
| Talc | 1 mg |
| Total | 150 mg |

The active ingredient, starch, and cellulose are passed through a No. 45 mesh U.S. sieve, and (0.355 mm) mixed thoroughly. The aqueous solution containing polyvinylpyrrolidone is mixed with the resultant powder, and the admixture then is passed through a No. 14 mesh U.S. sieve (1.4 mm). The granules so produced are dried at 50°C and passed through a No. 18 mesh U.S. sieve (1.0 mm). The sodium carboxymethyl starch, magnesium stearate, and talc, previously passed through No. 60 mesh U.S. sieve (0.250 mm), are then added to the granules which, after mixing, are compressed on a tablet machine to yield tablets each weighing 150 mg.

### Formulation Example 5

Capsules, each containing 80 mg of active ingredient, are made as follows:

| | |
|---|---|
| Active ingredient | 80 mg |
| Starch | 59 mg |
| Microcrystals cellulose | 59 mg |
| Magnesium stearate | 2mg |
| Total | 200 mg |

The active ingredient, cellulose, starch, and magnesium stearate are blended, passed through a No. 45 mesh U.S. sieve (0.355 mm), and filled into hard gelatin capsules in 200 mg quantities.

### Formulation Example 6

Suppository, each containing 225 mg of active ingredient, are made as follows:

| | |
|---|---|
| Active ingredient | 225 mg |
| Saturated fatty acid glycerides | 2000 mg |
| Total | 2225 mg |

The active ingredient is passed through a No. 60 mesh U.S. sieve (0.251 mm) and suspended in the saturated fatty acid glycerides previously melted using the minimum heat necessary. The mixture is then poured into a suppository mold of nominal 2g capacity and allowed to cool.

### Formulation Example 7

Suspensions, each containing 50 mg of active ingredient per 5 mL dose, are made as follows:

| | |
|---|---|
| Active ingredient | 50 mg |
| Sodium carboxymethyl cellulose | 50 mg |
| Syrup | 1.25 mL |
| Benzoic acid solution | 0.10 mL |
| Flavor | q.v. |
| Color | q.v. |
| Purified water to total | 5 mL |

The active ingredient is passed through a No. 45 U.S. sieve (0.355 mm), and mixed with the sodium carboxymethyl cellulose and syrup to form a smooth paste. The benzoic acid solution and flavor are diluted with a portion of the water and added, with stirring. Sufficient water is then added to produce the required volume.

### Formulation Example 8

An intravenous formulation may be prepared as follows:

| | |
|---|---|
| Active ingredient | 100 mg |
| Saturated fatty acid glycerides | 1000 mL |

The solution of the above ingredients generally is administered intravenously to a subject at a rate of 1 mL per minute.

### Industrial Applicability

It is provided that the type X sPLA₂ inhibitors of claim 1 are useful in preventing or treating cancer.

## Claims

1. Use of a type-X sPLA₂ inhibitor for the preparation of a medicament for the prevention or treatment of cancer, wherein the type-X sPLA₂ inhibitor is a compound represented by the formula: 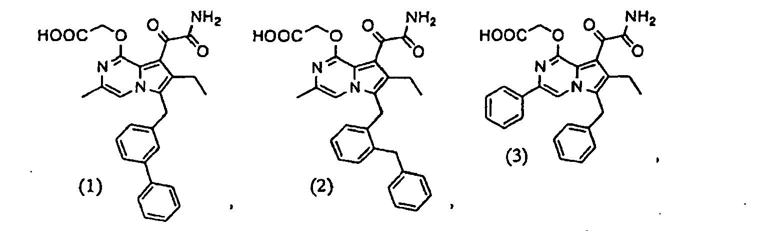 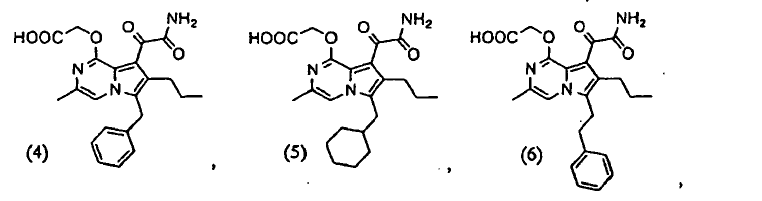 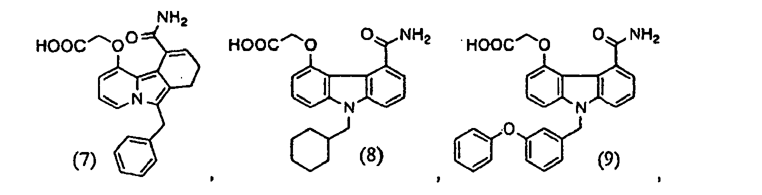 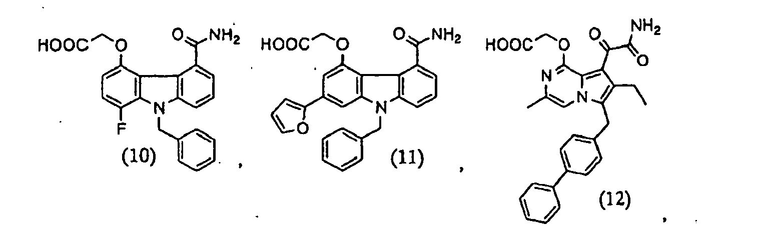 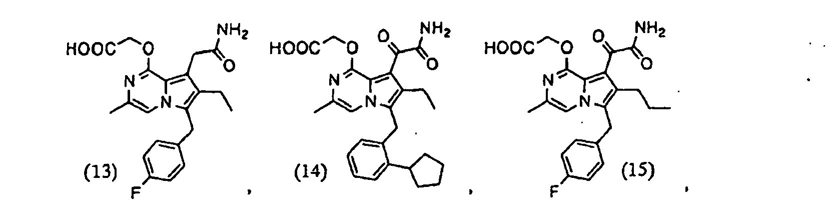 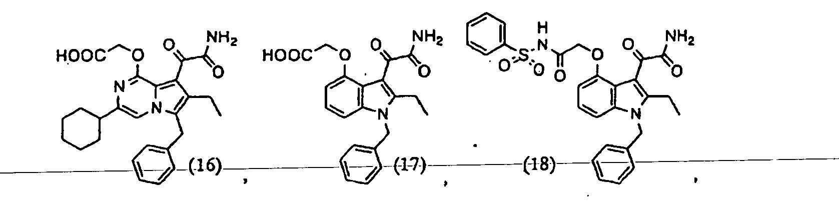 or 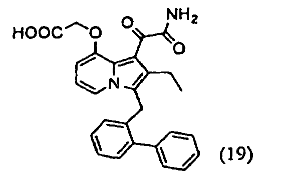 a C1-C6 alkyl ester thereof, its pharmaceutically acceptable salt, or its solvate.

2. Use as claimed in claim 1, wherein the cancer is colon cancer, lung cancer, liver cancer, gastric cancer, renal cancer, gallbladder cancer, prostatic cancer, pancreatic cancer, testis cancer, ovary cancer or cutaneous cancer.

3. Use as claimed in claim 1, wherein the cancer is colon cancer.

## Patentansprüche

1. Verwendung eines Typ-X sPLA₂-Inhibitors zur Herstellung eines Medikaments zur Vorbeugung oder Behandlung von Krebs, wobei der Typ-X sPLA₂-Inhibitor eine Verbindung der Formel: 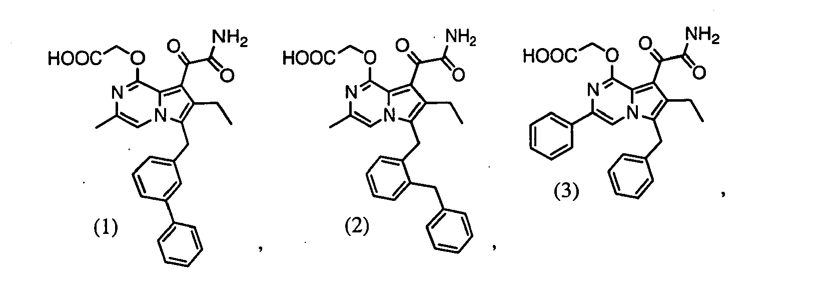 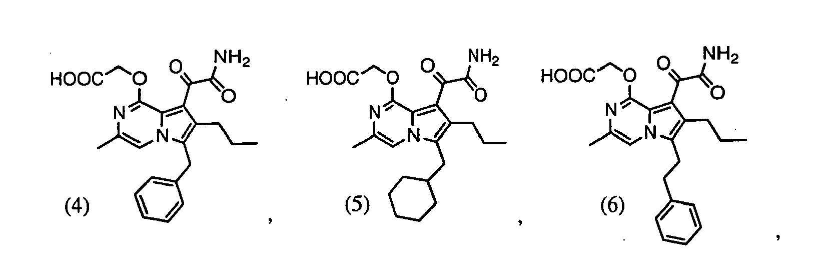 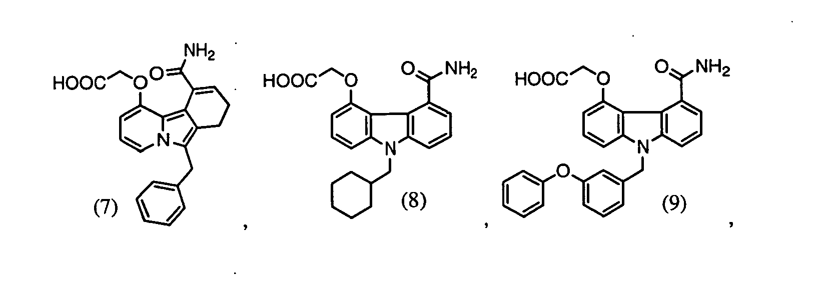 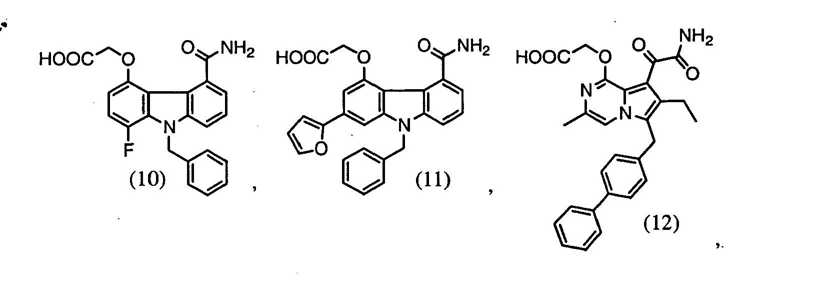 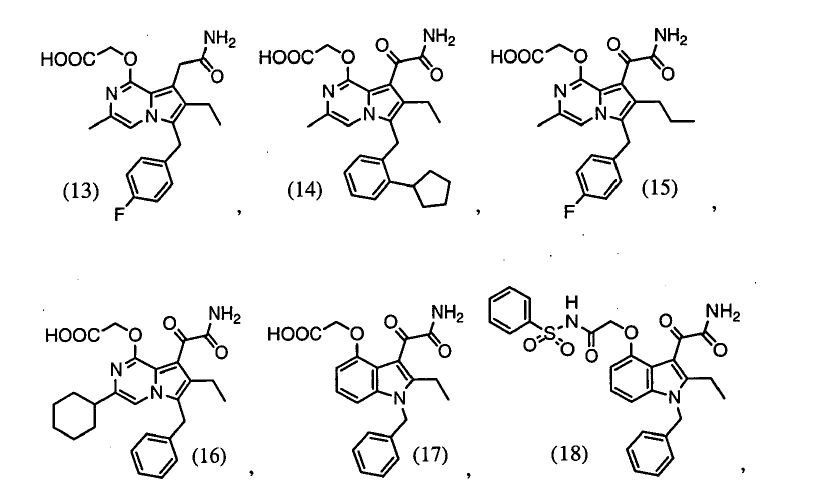 oder 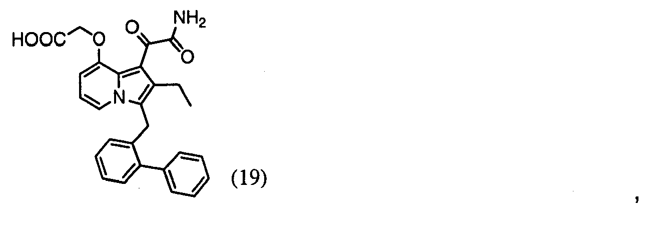 ein C1-C6 Alkylester davon, dessen pharmazeutisch verträgliches Salz oder dessen Solvat, ist.

2. Verwendung wie in Anspruch 1 beansprucht, wobei der Krebs Darmkrebs, Lungenkrebs, Leberkrebs, Magenkrebs, Nierenkrebs, Gallenblasenkrebs, Prostatakrebs, Pankreaskrebs, Hodenkrebs, Eierstockkrebs oder Hautkrebs ist.

3. Verwendung wie in Anspruch 1 beansprucht, wobei der Krebs Darmkrebs ist.

## Revendications

1. Utilisation d'un inhibiteur des sPLA₂ de type X pour la préparation d'un médicament pour la prévention ou le traitement du cancer, dans laquelle l'inhibiteur des sPLA₂ de type X est un composé représenté par la formule : 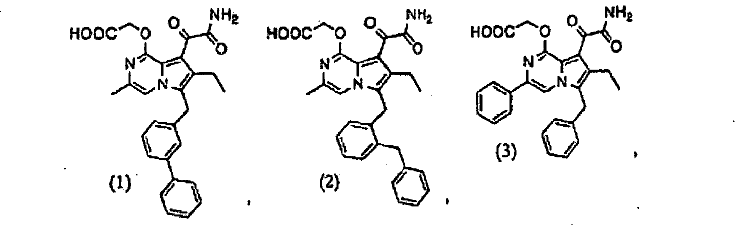 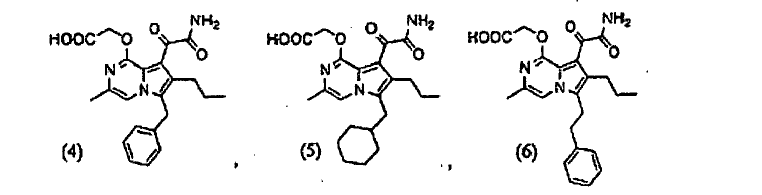 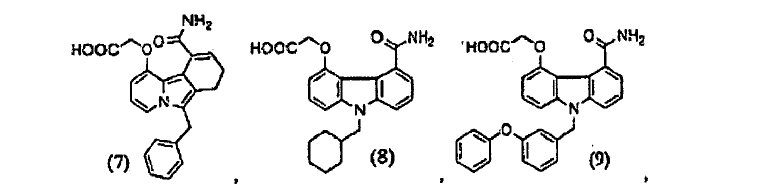 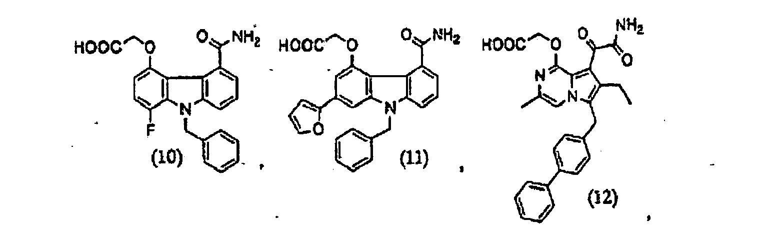 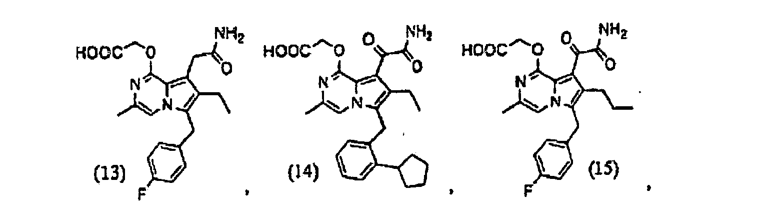 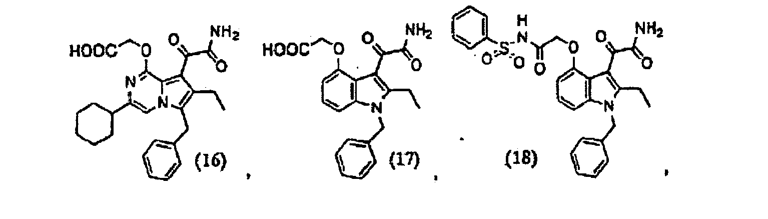 ou 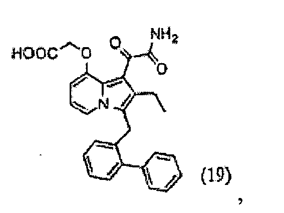 un ester d'alkyle en C₁-C₆ de ceux-ci, leur sel pharmaceutiquement acceptable, ou leur solvate.

2. Utilisation telle que revendiquée dans la revendication 1, dans laquelle le cancer est un cancer du colon, un cancer des poumons, un cancer du foie, un cancer de l'estomac, un cancer du rein, un cancer de la vésicule biliaire, un cancer de la prostate, un cancer du pancréas, un cancer des testicules, un cancer des ovaires, ou un cancer de la peau.

3. Utilisation telle que revendiquée dans la revendication 1, dans laquelle le cancer est un cancer du colon.
